(19) Europäisches Patentamt
European Patent Office
Office européen des brevets

(11) Publication number: 0 226 282 B1

(12) # EUROPEAN PATENT SPECIFICATION

(45) Date of publication of the patent specification: 05.07.89

(51) Int. Cl.[4]: C07D 487/04, A61K 31/505, C07D 271/06 // (C07D487/04, 239:00, 235:00)

(21) Application number: 86307541.2

(22) Date of filing: 01.10.86

(54) **Heterocyclic compounds and their preparation and use.**

(30) Priority: 17.10.85 DK 4768/85
17.10.85 DK 4769/85

(43) Date of publication of application: 24.06.87 Bulletin 87/26

(45) Publication of the grant of the patent: 05.07.89 Bulletin 89/27

(84) Designated Contracting States: AT BE CH DE ES FR GB GR IT LI LU NL SE

(56) References cited:
EP-A- 0 109 921
US-A- 4 374 988

JOURNAL OF ORGANIC CHEMISTRY, vol. 41, no. 4, February 20, 1976, pp. 620-626, Easton, Pa., US, J.E. FRANZ et al.: "Periselective addition of nitrile sulfides, nitrile oxides, and diphenyldiazomethane to tetracyanoethylene"

(73) Proprietor: A/S FERROSAN, Sydmarken 5, DK-2860 Soeborg(DK)

(72) Inventor: Wätjen, Frank, Ravnehusvej 27, DK-3500 Vaerloese(DK)
Inventor: Engelstoft, Mogens, Mosegard Park 121, DK-3500 Vaerloese(DK)

(74) Representative: Perry, Robert Edward et al, GILL JENNINGS & EVERY 53-64 Chancery Lane, London WC2A 1HN(GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

**Description**

The present invention relates to therapeutically-active heterocyclic compounds, to a method of preparing them and to pharmaceutical compositions comprising them. The novel compounds are useful in psychopharmaceutical applications, e.g. in the treatment of central nervous system ailments, for example, as anticonvulsants or anxiolytics.

It is well known (Squires, R.F. and Braestrup, C., Nature (London) 266 (1977) 732–4) that specific sites in the central nervous systems of vertebrates exhibit a high specific affinity for binding 1,4- and 1,5-benzodi-azepines. These sites are called benzodiazepine receptors.

EP-A 0 109 921 discloses oxadiazolyl-imidazo[1,4]-benzodiazepines having benzodiazepine receptor activity.

It has now been found that novel quinazoline compounds have strong affinity for the benzodiazepine receptors which make them useful in psychopharmaceutical preparations. The novel quinazoline compounds have the general formula I

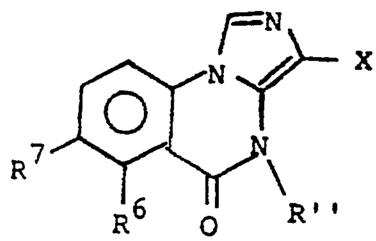

(I)

wherein

X is 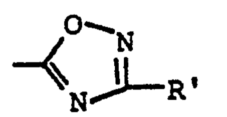 , 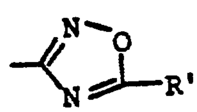 , or $CO_2R'$

wherein R' is $C_{1-6}$-alkyl, $C_{3-7}$-cycloalkyl, or $C_{3-7}$-cycloalkylmethyl,
$R^6$ and $R^7$ independently are hydrogen, halogen, alkoxy or trifluoromethyl , and
R" is hydrogen, $C_{1-6}$-alkyl, or $C_{3-7}$-cycloalkyl

The pharmaceutical properties of the compounds of the invention can be illustrated by determining their capability for displacing radioactivity labelled flunitrazepam from benzodiazepine receptors.

The displacement activity of the compounds of the invention may be found by determining the $ED_{50}$ value. The $ED_{50}$ value represents the dose (mg/kg) of a test substance which causes the specific binding of flunitrazepam to benzodiazepine receptors in a living brain to be reduced to 50% of the control value.

Such an in vivo test is carried out as follow:

Principle. Twenty minutes after a dose of $^3$H-flunitrazepam ($^3$H-FNM) (200µ Ci/kg, i.v.) the amount of specific $^3$H-FNM binding to brain benzodiazepine receptors has reached its maximal value. This specific binding of $^3$H-FNM can be partly or completely prevented by simultaneous or prior administration of pharmacologically active benzodiazepines and by some benzodiazepine-like agents (Chang and Snyder, Eur.J. Pharmacol. 48, 212-218 (1978).

Test procedure. Suspensions of test substances (2 mg/ml) are prepared in 5% Duphasol-X® (TM Duphar, castor oil-ethylene oxide derivative for emulsifying and solubilizing oil and other water-insoluble substances) by sonification for 10 min using a Branson® B15 microtip ultrasonifier (setting 7). Groups of three mice (female, NMR, 18-22 gram) are injected with the test substance at 100 mg/kg intraperitoneally. Fifteen minutes after test substance administration the mice are challenged with 4µ Ci intravenously of $^3$H-FNM (70-90 Ci/mole) in 200 µl physiological saline. Twenty minutes after $^3$H-FNM administration mice are sacrificed by decapitation, the forebrains rapidly excised (within 30 sec) and homogenized in 12 ml of icecold 25 mM $KH_2PO_4$, pH 7.1, using an Ultra-Turrax® homogenizer fitted with an N 10 shaft. Two aliquots of 1 ml are immediately filtered through Whatman® GF/C glassfibre filters and washed with 2 x 5 ml of the above mentioned buffer. The amounts of radioactivity on the filters are determined by conventional scintillation counting. One group of untreated mice serves as controls. One to three mice are injected with 25 g/kg clonazepam i.p. 30 minutes before $^3$H-FNM to determine the amount of non-specific $^3$H-FNM binding, which should be between 8-15% of total binding. When doses of 100 mg/kg inhibit more than 50% of specific $^3$H-flunitrazepam binding, test substances are administered in doses, which are factors of 3.16 times lower than 100 mg/kg. The $ED_{50}$ for a test substance is defined as that dose which inhibits 50% of specific $^3$H-FNM binding. Specific binding is the amount of binding in controls minus the amount binding in clonazepam-treated mice.

Results. The $ED_{50}$ value is determined from dose response curves. If only one dose of test sub-

stance is administered the $ED_{50}$ value is calculated as follows, provided that the inhibition of specific binding is within the range of 25-75%:

$$ED_{50} = (\text{administered dose}) \times \frac{1}{\left(\frac{C_o}{C_x} - 1\right)} \text{ mg/kg}$$

where $C_o$ is specific binding in controls and $C_x$ is specific binding in mice treated with test substance.

Test results obtained by testing some compounds of the invention will appear from the following table I.

**TABLE 1.**

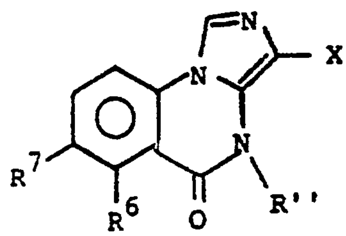

| $R^6$ | $R^7$ | R'' | X | in vivo $ED_{50}$ (mg/kg |
|---|---|---|---|---|
| Cl | H | $CH_3$ | 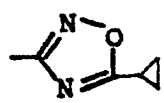 | 0,69 |
| Cl | H | $CH_3$ | 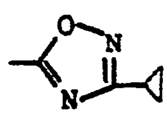 | 0,41 |
| $OC_2H_5$ | H | $CH_3$ | 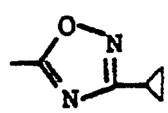 | 2,1 |
| H | H | $C_2H_5$ | 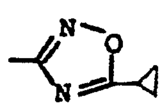 | 3,5 |
| Cl | H | $C_2H_5$ | $CO_2C_2H_5$ | 4,1 |
| $CF_3$ | H | $CH_3$ | 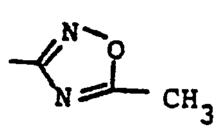 | 5,6 |

The invention also relates to a method of preparing the above mentioned compounds. This method comprises:

a) reacting a compound of formula II

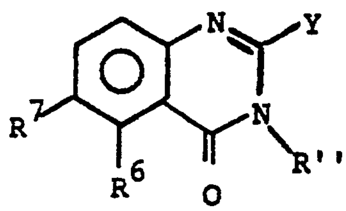

(II)

wherein R",$R^6$ and $R^7$ have the meanings set forth above and wherein Y is a leaving group, with a compound having the formula III
CN - $CH_2$ - X (III)
wherein X has the meaning set forth above, to form a compound of the invention. Compounds of formula III are described and claimed in our copending application No. 87 114 729.4 (EP-A 0 274 009)

b) reacting a reactive derivative of a compound having the general formula IV

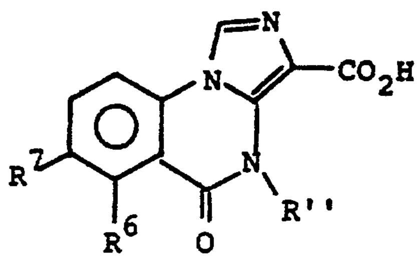

(IV)

wherein R",$R^6$ and $R^7$ have the meanings set forth above, with a compound having the general formula V

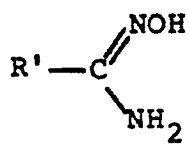

(V)

wherein R' has the meanings set forth above to form a compound of the general formula I wherein X is

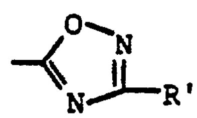

wherein R' has the meaning set forth above,

c) reacting a compound having the general formula VI

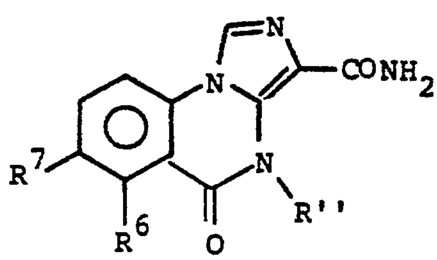

(VI)

wherein R",$R^6$ and $R^7$ have the meanings set forth above, with a compound having the general formula VII
R'-$C(OCH_3)_2N(CH_3)_2$ (VII)
wherein R' has the meaning set forth above to form a compound having the general formula VIII

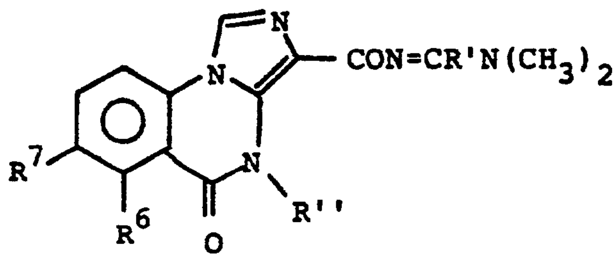

(VIII)

wherein R',R",$R^6$ and $R^7$ have the meanings set forth above and reacting the compound having the formula (VIII) with $NH_2OH$ or another aminating agent to form a compound having the generic formula I, wherein X is

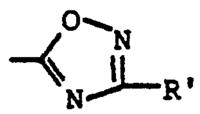

wherein R' has the meaning defined above;

d) reacting a compound having the general formula IX

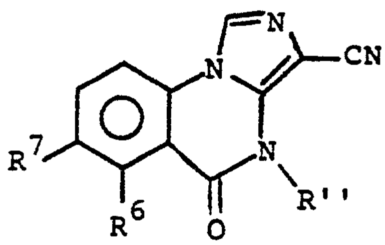

(IX)

wherein R",$R^6$ and $R^7$ have the meanings set forth above, with $NH_2OH$ to form a compound having the general formula X

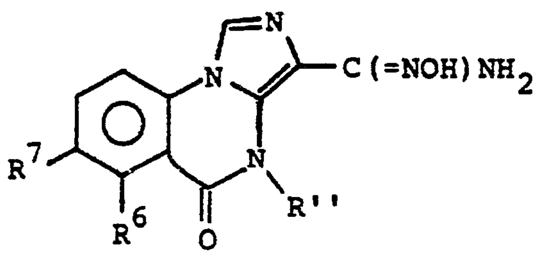

(X)

wherein R",$R^6$ and $R^7$ have the meanings set forth above and reacting the compound having the formula (X) with R'-COCl, wherein R' has the meaning set forth above, to form a compound of formula I, wherein X is

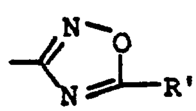

wherein R' has the meaning set forth above.

The leaving group, Y, may be any and for example those disclosed in U.S. Patents 4,031,079 or 4,359,420, for example, halogen, alkylthio, e.g., methylthio, aralkylthio, N-nitrosoalkylamino, alkoxy, mercapto, $-OP(O)(OR)_2$ wherein R is lower-alkyl or -OP(O)(NR`R``) wherein R` and R`` each represents lower-alkyl or phenyl, or together with the nitrogen atom to which they are attached represent a heterocyclic radical such as morpholino, pyrrolidino, piperidino, or methylpiperazino. The reaction is preferably carried out under alkaline conditions, i.e., in the presence of a base, and among bases alkali metal, e.g., potassium or sodium, alkoxides or hydrides are preferred. The reaction is preferably conducted in the presence of an organic solvent which is nonreactive with the reactants and products of reaction under the conditions of reaction, especially an anhydrous solvent and preferably an anhydrous aprotic solvent such as dimethylformamide (DMF) or the like. The temperature range employed may be any range

suitable for the reaction to proceed at a reasonable rate and without undue delay or decomposition and a range from a minus forty (-40) degrees Celsius to about room temperature is accordingly usually particularly suitable.

The starting materials may be prepared from commercially available benzene derivatives and by using well known synthetic methods and as described in Synthesis, Vol. 10, pp. 681-2.

The compound of the invention, together with a conventional adjuvant, carrier, or diluent, and if desired in the form of a pharmaceutically-acceptable acid addition salt thereof, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective central nervous system ailment alleviating amount of the active ingredient commensurate with the intended daily dosage range to be employed. Tablets containing ten (10) milligrams of active ingredient or, more broadly, ten (10) to thirty (30) milligrams, per tablet, are accordingly suitable representative unit dosage forms.

The compound of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or oral application which do not deleteriously react with the active compound.

Examples of such carriers are water, salt solutions, alcohols, polyethylene glycols, polyhydroxyethoxylated castor oil, gelatin, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxilliary agents, such as lubricants, preservatives, stabilizers, wetting agents, emulsifiers, salt for influencing osmotic pressure, buffers and/or coloring substances and the like, which do not deleteriously react with the active compound.

For parenteral application, particularly suitable are injectable solutions or suspensions, preferably aqueous solutions with the active compound dissolved in polyhydroxylated castor oil.

Ampoules are conveniently unit dosages.

For oral application, particularly suitable are tablets, dragees, or capsules having talc and/or a carbohydrate carrier or binder or the like, the carrier preferably being lactose and/or corn starch and/or potato starch. A syrup, elixir or like can be used when a sweetened vehicle can be employed. Generally, as to broader ranges, the compound of the invention is dispensed in unit dosage form comprising 0.05-100 mg in a pharmaceutically-acceptable carrier per unit dosage.

A typical tablet which may be prepared by conventional tabletting techniques contains:

| | |
|---|---|
| Active compound | 1.0 mg |
| Lactosum | 67.8 mg Ph.Eur. |
| Avicel® | 31.4 mg |
| Amberlite® IRP 88 | 1.0 mg |
| Magnesii stearas | 0.25 mg Ph. Eur. |

Due to their high degree of affinity for the benzodiazepin receptors, the compounds of the invention are extremely useful in the treatment of central nervous system ailments or disorders, when administered in an amount effective for the alleviation, amelioration, or elimination thereof. The important CNS activity of the compound of the invention includes both anticonvulsant and anxiolytic activities along with a low toxicity, together presenting a most favorable therapeutic index. The compound of the invention may accordingly be administered to a subject, e.g., a living animal body, including a human, in need of the same for the treatment, alleviation, amelioration, or elimination of an indication, associated with the central nervous system and the socalled benzodiazepin receptors, which requires such psychopharmaceutical treatment, e.g., especially convulsion and/or anxiety states, if desired in the form of a pharmaceutically-acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultaneously, or together with a pharmaceutically-acceptable carrier or diluent, especially and preferably in the form of a pharmaceutical composition thereof, whether by oral, rectal, or parenteral (including subcutaneous) route, in an effective psychopharmaceutical central nervous system ailment alleviating amount, e.g., an anticonvulsant and/or anxiolytic amount, and in any event an amount which is effective for the alleviation of such a central nervous

system ailment due to their benzodiazepine receptor affinity. Suitable dosage ranges are 1-200 milligrams daily 10-100 milligrams daily, and especially 30-70 milligrams daily, depending as usual upon the exact mode of administration, form in which administered, the indication toward which the administration is directed, the subject involved and the body weight of the subject involved, and the preference and experience of the physician or veterinarian in charge. Broader ranges for dosages of the compounds according to this invention are 0.1-300 mg/day, preferably 1-30 mg/day, when administered to patients, e.g., humans, as a drug.

The invention will now be described in further detail with reference to the following examples:

EXAMPLE 1

A. Isatoic anhydride

7.5 g of 2-amino benzoic hydrochloride was mixed with 10 ml of diphosgen and the mixture was stirred in 150 ml of dioxan for 40 minutes at reflux. The resulting mixture was cooled and filtered. Yield: 5.7 of title compound.

In the same manner, from the appropriate aminobenzoic acids the following compounds were synthesized:
6-fluoroisatoic anhydride
6-chloroisatoic anhydride
6-bromoisatoic anhydride
6-trifluromethylisatoic anhydride
5-chloroisatoic anhydride
5-fluoroisatoic anhydride

B. 1,2,3,4-tetrahydro-3-methyl-2,4-dioxo-quinazoline

6 g isatoic anhydride was stirred in 100 ml dry tetrahydrofuran (THF) and methylamine was passed through the mixture for 5 min. The resulting solution was evaporated and the residue was again dissolved in THF (100 ml) and charged with 15 ml 30% phosgen solution in toluene. The mixture was heated to reflux and additional 15 ml phosgen solution was added. After 4 hours reflux the mixture was cooled and evaporated to dryness. The residue was treated with water and the crystals were collected by filtration. The yield was 3.5 g. M.p. 240.4-240, 5°C.

In the same manner, from the appropriate substituted isatoic anhydrides the following compounds were synthesized:
3-methyl-5-chloro-2,4-dioxo-1,2,3,4-tetrahydro-quinazoline. M.p. 226-9°C.
3-methyl-5-bromo-2,4-dioxo-1,2,3,4-tetrahydro-quinazoline. M.p. 280°C.
3-methyl-6-chloro-2,4-dioxo-1,2,3,4-tetrahydro-quinazoline.
3-methyl-6-fluoro-2,4-dioxo-1,2,3,4-tetrahydro-quinazoline.
3-methyl-5-fluoro-2,4-dioxo-1,2,3,4-tetrahydro-quinazoline.
3-methyl-5-trifluoromethyl-2,4-dioxo-1,2,3,4-tetrahydroquinazoline.

C. 3-methyl-5-iodo-2,4-dioxo-1,2,3,4-tetrahydro-quinazoline

1.81g of 3-methyl-5-bromo-2,4-dioxo-1,2,3,4-tetrahydroquinazoline, 11.8g of potassium iodide and 6.76g of cupro iodide were stirred in 40ml of HMPA (hexamethylphosphoramide) at 150°C under nitrogen for 7 hours. The mixture was then left at room temperature overnight. Next day 40 ml 1M hydrochloric acid, 40 ml water and 50 ml methylene chloride were added. The combined mixture was filtered and the methylene chloride phase of the filtrate was evaporated in vacuo to give 1.3g of residue. The residue was recrystallized from acetone. Yield: 0.77g of the title compound. M.p. >300°C.

D. 3-cyclopropyl-5-isocyanomethyl-1,2,4-oxadiazole

a. 3-cyclopropyl-5-formylaminomethyl-1,2,4-oxadiazole.

A solution of ethyl formylaminomethyl-carboxylate (150 mmol) and cyclopropyl carboxamide oxime (100 mmol) in 100% EtOH (100 ml) was charged with Na (200 mg) and a crushed molecular sieve (4Å) (10 g). The mixture thus obtained was stirred and heated to reflux for 8 hours. The mixture was cooled to room temperature, filtered through filter aid and the filtrate was evaporated in vacuo. The oily residue was partitionated into a $CHCl_3$ phase which was dried with $Na_2SO_4$ and evaporated.

b. 3-cyclopropyl-5-isocyanomethyl-1,2,4-oxadiazole.

A stirred solution of 3-cyclopropyl-5-formylaminomethyl-1,2,4-oxadiazole (60 mmol) and triethylamine

(176 mmol) in $CH_2Cl_2$ (100 ml) was charged dropwise with $POCl_3$ (60 mmol) at 0°C. The mixture was then left for 30 minutes with stirring at 0°C, whereafter a solution of $Na_2CO_3$ (60 mmol) in $H_2O$ (50 ml) was added. The mixture was heated to room temperature, whereafter the organic phase was separated, dried and evaporated in vacuo. The residue was treated with ether, decanted and the solution was evaporated to give the title compound as an oil. The oil was processed without any further purification. The compound was characterized by its IR absorption band at 2160 $cm^{-1}$.

3-ethyl-5-isocyanomethyl-1,2,4-oxadiazole was prepared from 3-ethyl-5-formylaminomethyl-1,2,4-oxadiazole in a similar manner. IR: $cm^{-1}$: 2170.

Our copending Application No. 87 114 729.4 (EP-A 0 274 009) relates to the preparation of other compounds exemplifying formula III, i.e.
5-cyclobutyl-3-isocyanomethyl-1,2,4-oxadiazole
5-ethyl-3-isocyanomethyl-1,2,4-oxadiazole
5-methyl-3-isocyanomethyl-1,2,4-oxadiazole
5-methoxymethyl-3-isocyanomethyl-1,2,4-oxadiazole

E. 3-(5-ethyl-1,2,4-oxadiazol-3-yl)-4,5-dihydro-4-methyl-5-oxoimidazo(1,5-a)quinazoline

3-methyl-1,2,3,4-tetrahydro-2,4-dioxoquinazoline (5 mmol) was dissolved in dry dimethylformamide (20 ml) and charged with sodium hydride (6 mmol). The resulting solution was cooled under $N_2$ to –20°C, whereafter chlorodiethylphosphate (6 mmol) was added. The reaction mixture was kept under $N_2$ with stirring, was allowed to reach room temperature, and was then charged with a cold (–30°C) solution of 5-ethyl-3-isocyanomethyl-1,2,4-oxadiazole (6 mmol) and potassium t-butylate (6 mmol) in dry DMF (15 ml). The resulting mixture was stirred at room temperature for one hour whereafter the reaction mixture was evaporated in vacuo. The residue was partitioned between ethyl acetate and 4M sodium hydroxide. The organic phase was dried and evaporated. Yield 120 mg of title compound. M.p. 199.4-202.2°C.

In the same manner the following compounds were synthesized:

Ethyl 4,5-dihydro-4-methyl-5-oxo-imidazo(1,5-a)quinazoline-3-carboxylate. M.p. 211.4-211.8°C by reaction between 3-methyl-1,2,3,4-tetrahydro-2,4-dioxo-quinazoline and ethyl isocyanoacetate.

Ethyl 4,5-dihydro-4-methyl-5-oxo-6-chloro-imidazo(1,5-a)-quinazoline-3-carboxylate. M.p. 248-54°C by reaction between 3-methyl-5-chloro-1,2,3,4-tetrahydro-2,4-dioxo-quinazoline and ethyl isocyanoacetate.

Ethyl 4,5-dihydro-4-methyl-5-oxo-6-bromo-imidazo(1,5-a)-quinazoline-3-carboxylate. M.p. 272°C by reaction between 3-methyl-5-bromo-1,2,3,4-tetrahydro-2,4-dioxo-quinazoline and ethyl isocyanoacetate.

3-(5-cyclopropyl-1,2,4-oxadiazol-3-yl)-4,5-dihydro-4-methyl-5-oxo-6-chloro-imidazo(1,5-a)quinazoline. M.p. 230-40°C (decomp.) by reaction between 3-methyl-5-chloro-1,2,3,4-tetrahydro-2,4-dioxo-quinazoline and 5-cyclopropyl-3-isocyano-methyl-1,2,4-oxadiazole.

3-(5-cycloporopyl-1,2,4-oxadiazol-3-yl)-4,5-dihydro-4-methyl-5-oxo-6-bromo-imidazo(1,5-a)quinazoline. M.p. 206.6°C by reaction between 3-methyl-5-bromo-1,2,3,4-tetrahydro-2,4-dioxo-quinazoline and 5-cyclopropyl-3-isocyanomethyl-1,2,4-oxadiazole.

3-(5-ethyl-1,2,4-oxadiazol-3-yl)-4,5-dihydro-4-methyl-5-oxo-7-chloro-imidazo(1,5-a)quinazoline. M.p. 196-8°C. by reaction between 3-methyl-6-chloro-1,2,3,4-tetrahydro-2,4-dioxo-quinazoline and 5-ethyl-3-isocyanomethyl-1,2,4-oxadiazole.

3-(5-cyclopropyl-1,2,4-oxadiazol-3-yl)-4,5-dihydro-4-methyl-5-oxo-6-iodo-imidazo(1,5-a)quinazoline. M.p. 217-20°C by reaction between 3-methyl-5-iodo-1,2,3,4-tetrahydro-2,4-dioxo-quinazoline and 5-cyclopropyl-3-isocyanomethyl-1,2,4-oxadiazole.

Ethyl 4,5-dihydro-4-methyl-5-oxo-6-iodo-imidazo(1,5-a)-quinazoline-3-3-carboxylate. M.p. 266-67°C by reaction between 3-methyl-5-iodo1,2,3,4-tetrahydro-2,4-dioxo-quinazoline and ethyl isocyanoacetate.

3-(5-cyclopropyl-1,2,4-oxadiazol-3-yl)-4,5-dihydro-4-methyl-5-oxo-imidazo(1,5-a)quinazoline. M.p. 206.7-207.8°C by reaction between 3-methyl-1,2,3,4-tetrahydro-2,4-dioxoquinazoline and 5-cyclopropyl-3-isocyanomethyl-1,2,4-oxadiazole.

3-(5-methyl-1,2,4-oxadiazol-3-yl)-4,5-dihydro-4-methyl-5-oxo-6-trifluoromethyl-imidazo(1,5-a)quinazoline, M.p. 251.5-251.9°C, by reaction between 3-methyl-5-trifluromethyl-1,2,3,4-tetrahydro-2,4-dioxo-quinazoline and 5-methyl-3-isocyanomethyl-1,2,4-oxadiazole.

3-(3-cyclopropyl-1,2,4-oxadiazol-5-yl)-4,5-dihydro-4-methyl-5-oxo-6-bromo-imidazo(1,5-a)quinazoline. M.p. 246-47°C by reaction between 3-methyl-5-bromo-1,2,3,4-tetrahydro-2,4-dioxoquinazoline and 3-cyclopropyl-5-isocyanomethyl-1,2,4-oxadiazole.

EXAMPLE 2.

3-(5-cyclopropyl-1,2,4-oxadiazol-3-yl)-4,5-dihydro-4-methyl-5-oxo-6-ethoxy-imidazo(1,5-a)quinazoline

30 mg of sodium was dissolved in 10 ml of dry ethanol. Then 3-(5-cyclopropyl-1,2,4-oxadiazol-3-yl)-4,5-dihydro-4-methyl-5-oxo-6- chloro-imidazo(1,5-a)quinazoline (0.2 g, 0.5 mmol) was added and the resulting mixture was refluxed for 7 hours. The reaction mixture was evaporated. Glacial acetic acid and water was added to the residue whereupon the title compound was isolated by filtration. M.p. 195-205°C.

EXAMPLE 3.

A. Methoxyacetamide oxime

2.3 g of sodium in 33 ml of dry methanol was mixed with 6.55 g of hydroxylamine hydrochloride in 66 ml of dry methanol. The mixture was filtered and 7.8 g of methoxyacetonitrile was added dropwise to the filtrate. The mixture was left for 48 hours. The mixture was then cooled to 4°C. Filtration and evaporation of the filtrate give 8.7 g of the title compound.

The following compounds were synthesized from the appropriate nitrites in an analogous manner:
Propionamide oxime
Cyclopropyl carboxamide oxime
Isopropyl carboxamide oxime

B. 3-(3-cyclopropyl-1,2,4-oxadiazol-5-yl)-4,5-dihydro-4-methyl-5-oxo-6-chloro-imidazo(1,5-a)quinazoline and 3-(3-cyclopropyl-1,2,4-oxadiazol-5-yl)-4,5-dihydro-4-methyl-5-oxo-6-ethoxy-imidazo(1,5-a)quinazoline

50 mg of sodium was dissolved in 20 ml of dry ethanol containing 3 g of a molecular sieve (4Å) and 0.5 g of cyclopropylcarboxamide oxime was added to this mixture and thereupon 0.2 g of ethyl 4,5-dihydro-4-methyl-5-oxo-6-chloro-imidazo-(1,5-a)quinazoline-3-carboxylate. The resulting mixture was refluxed for 2 hours. The product was isolated by filtration, reduction of the volume of the reaction mixture in vacuo followed by addition of icewater and filtration. T.L.C. showed two compounds, which were isolated by chromatography on silica gel with ethyl acetate.

Yield: 5.7mg of 6-chloro compound M.p. 200-5°C.
5.0mg of 6-ethoxy compound M.p. 238-40°C.

3-(3-isopropyl-1,2,4-oxadiazol-5-yl)-4,5-dihydro-4methyl-5-oxo-6-chloro-imidazo(1,5-a)quinazoline, M.p.208,7-209,3°C was prepared in exactly the same manner from isopropyl carboxamide oxime.

EXAMPLE 4.

6-cyano-3-(5-cyclopropyl-1,2,4-oxadiazol-3-yl)-4,5-dihydro-4-methyl-5-oxo-imidazo(1,5-a)quinazoline.

To 140 mg 6-bromo-3-(5-cyclopropyl-1,2,4-oxadiazol-3-yl)-4,5-dihydro-4-methyl-5-oxo-imidazo(1,5-a)quinazoline in 5 ml dimethyl formamide (DMF) 40 mg cupro cyanide was added. Thereafter further 5 ml DMF was added and the resulting mixture was heated to 130-50°C for 60 minutes with stirring. To this mixture 110 mg sodium cyanide in 5 ml water was added and thereafter further 30 ml water. The resulting mixture was extracted with 30 ml ethyl acetate and thereafter four times with 20 ml ethyl acetate. The combined organic phase was washed with water and dried with calcium chloride. Evaporation in vacuo gave 24 mg of the title compound. M.p. 115-25°C.

EXAMPLE 5.

A. 1,2,3,4-tetrahydro-3-ethyl-2,4-dioxo-quinazoline

To a mixture of 12,23 g of ethylamine hydrochloride , 35 ml 4M sodium hydroxide and 175 ml methylene chloride was added 16,3 g isatoic anhydride. This mixture was stirred for 4 hours and the aqueous phase was made basic with 4 M sodium hydroxide. The organic phase was evaporated in vacuo. The residue was dissolved in 300 ml of tetrahydrofuran (THF) and 75 ml phosgene solution (20% in toluene) was added. The resulting mixture was stirred at 80°C for two hours. The precipitate was filtered of and the mother liquor was evaporated leaving 5.2 g of the title compound. M.p. 189.8-189.9°C.

In exactly the same manner the following compounds were prepared:
1,2,3,4-tetrahydro-3-cyclopropyl-2,4-dioxo-5-chloro-quinazoline, M.p. 284.8-285.0°C, from cyclopropylamine and 6-chloroisatoic anhydride.
1,2,3,4-tetrahydro-3-ethyl-2,4-dioxo-5-chloro-quinazoline, M.p. 267.2-267.5°C, from ethylamine and 6-chloroisatoic anhydride.

B. 3-(5-cyclopropyl-1,2,4-oxadiazol-3-yl)-4,5-dihydro-4-ethyl-5-oxo-imidazo(1,5-a)quinazoline

A mixture of 1.9 g 1,2,3,4-tetrahydro-3-ethyl-2,4-dioxoquinazoline and 450 mg sodium hydride in 50 ml of dry dimethyl formamide (DMF) was cooled to -20°C. Under an atmosphere of nitrogen was added 1.7 ml chlorodiethylphosphate. To this mixture was added a -30°C cold solution of 1.3 g K-t-butoxide and 2.2 g 3-isocyanomethyl-5-cyclopropyl-1,2,4-oxadiazole in 20 ml of dry DMF. The resulting mixture was stirred for 20 minutes whereafter 1 ml acetic acid/50 ml water/20 ml diethyl ether was added which caused the product to precipitate. Yield 0.6 g. M.p. 180-81°C.

The following compounds were prepared in exactly the same manner:

Ethyl 4,5-dihydro-4-ethyl-5-oxo-5-chloro-imidazo(1,5-a)quinazoline-3-carboxylate, M.p. 226.4-226.6°C, by reaction between 3-ethyl-1,2,3,4-tetrahydro-2,4-dioxo-5-chloro-quinazoline and ethyl isocyanoacetate.

Ethyl 4,5-dihydro-4-cyclopropyl-5-oxo-6-chloro-imidazo(1,5-a)-quinazoline-3-carboxylate, M.p. 207.0-210.5°C, by reaction between 3-cyclopropyl-1,2,3,4-tetrahydro-2,4-dioxo-5-chloro-quinazoline and ethyl isocyanoacetate.

3-(5-cyclopropyl-1,2,4-oxadiazol-3-yl-4,5-dihydro-4-ethyl-5-oxo-6-chloro-imidazo(1,5-a)quinazoline. M.p. 217.7-218.1°C by reaction between 3-ethyl-5-chloro-1,2,3,4-tetrahydro-2,4-dioxo-quinazoline and 5-cyclopropyl-3-isocyanomethyl-1,2,4-oxadiazole.

3-(5-cyclopropyl-1,2,4-oxadiazol-3-yl-4,5-dihydro-4-cyclopropyl-5-oxo-6-chloro-imidazo(1,5-a)quinazoline. M.p. 232- 35°C by reaction between 3-cyclopropyl -5-chloro-1,2,3,4-tetrahydro-2,4-dioxo-quinazoline and 5-cyclopropyl-3-iso cyanomethyl-1,2,4-oxadiazole.

EXAMPLE 6.

Methyl 4,5-dihydro-4-methyl-5-oxo-6-chloro-imidazo(1,5-a)-quinazoline-3-carboxylate.

1g of 4,5-dihydro-4-methyl-5-oxo-6-chloro-imidazo(1,5-a)quinoxaline-3-carboximidazolide ( conventionally prepared by reacting the free acid with N,N-carbonyldiimidazole) was refluxed in 20 ml of methanol at 60°C for 17 hours. The reaction mixture was eveporated in vacuo and the product was crystallized from water. Yield 0.82g of title compound. M.p. 210-220°C.

Isopropyl 4,5-dihydro-4-methyl-5-oxo-6-chloro-imidazo(1,5-a)-quinazoline-3-carboxylate, M.p. 215,7-219,5°C was prepared in exactly the same manner by refluxing in isopropanol.

Cyclopropylmethyl 4,5-dihydro-4-methyl-5-oxo-6-chloro-imidazo-(1,5-a)quinazoline-3-carboxylate, M.p. 214,2-214,3°C was prepared in exactly the same manner by refluxing in cyclopropylmethanol.

**Claims (for the designated states, BE, CH, DE, FR, GB, IT, LI, LU, NL, SE)**

1. A quinazoline compound having the formula

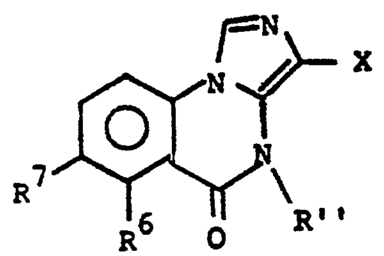

(I)

wherein

X is (i) 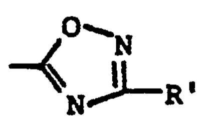 , (ii) 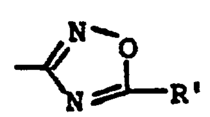 or (iii) $CO_2R'$

wherein R' is $C_{1-6}$ alkyl, $C_{3-7}$ cycloalkyl or $C_{3-7}$ cycloalkylmethyl,
$R^6$ and $R^7$ independently are hydrogen, halogen, alkoxy or trifluoromethyl, and
R" is hydrogen, $C_{1-6}$ alkyl or $C_{3-7}$ cycloalkyl.

2. A compound of claim 1, which is 3-(5-cyclopropyl-1,2,4-oxadiazol-3-yl)-4,5-dihydro-4-methyl-5-oxo-6-chloroimidazo(1,5-a)quinazoline.

3. A compound of claim 1 or claim 2, for therapeutic use.

4. Use of a compound of claim 1 or claim 2 for the manufacture of a medicament for use in the treatment of a disorder of the central nervous system.

5. A method of preparing a compound according to claim 1, which comprises reacting a compound of formula II

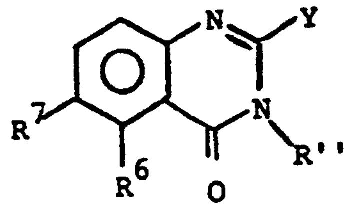

(II)

with a compound of formula III
CN-$CH_2$-X (III)
wherein R", $R^6$, $R^7$ and X are as defined in claim 1 and Y is a leaving group.

6. A method of preparing a compound according to claim 1 when X has definition (i), which comprises reacting a reactive derivative of a compound of formula IV

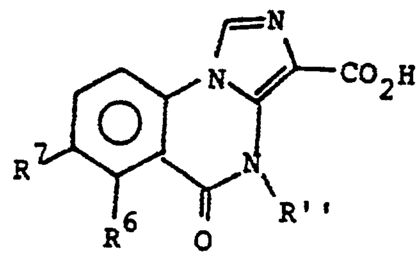

(IV)

with a compound of formula V
R'–C(=NOH)–$NH_2$ (V)
wherein R', R", $R^6$ and $R^7$ are as defined in claim 1.

7. A method of preparing a compound according to claim 1 when X has definition (i), which comprises reacting a compound of formula VI

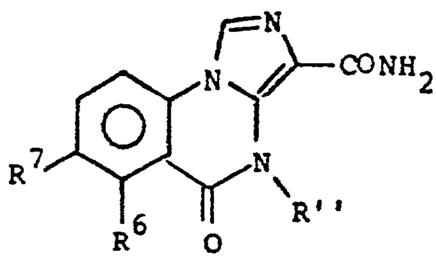

(VI)

with a compound of formula VII
R'–$C(OCH_3)_2$–$N(CH_3)_2$ (VII)
and reacting the resultant compound of formula VIII

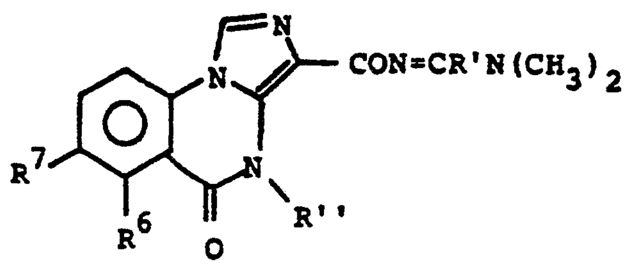

(VIII)

with $NH_2OH$ or another aminating agent, wherein R', R", $R^6$ and $R^7$ are as defined in claim 1.

8. A method of preparing a compound according to claim 1 when X has definition (ii), which comprises reacting a compound of formula IX

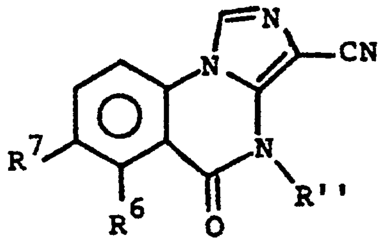

(IX)

with $NH_2OH$, and reacting the resultant compound of formula X

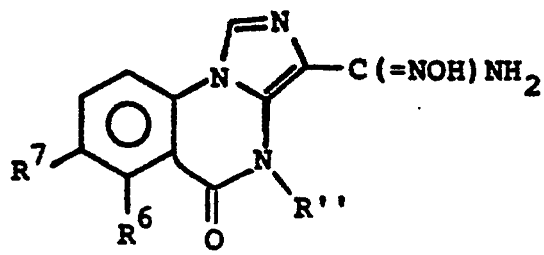

(X)

with R'-COCl, wherein R', R", $R^6$ and $R^7$ are as defined in claim 1.

9. A method according to claim 5, which is carried out under alkaline conditions.

10. A compound having the formula $CN-CH_2-Z$ wherein

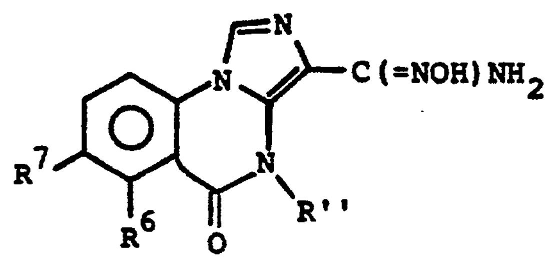

(X)

wherein R'" is $C_{1-6}$ alkyl or $C_{3-6}$ cycloalkyl and R"" is $C_{1-6}$ alkyl or $C_{4-6}$ cycloalkyl.

**Claims (for the Designated States : AT, ES, GR)**

1. Use of a quinazoline compound having the formula

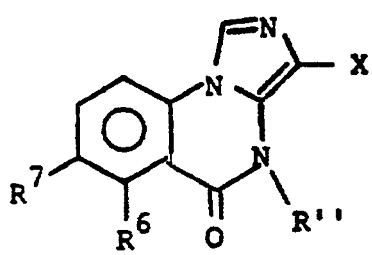

(I)

wherein

X is (i) 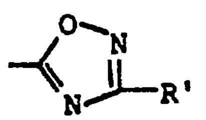 , (ii) 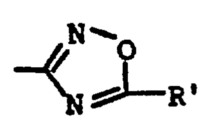 or (iii) $CO_2R'$

wherein R' is $C_{1-6}$ alkyl, $C_{3-7}$ cycloalkyl or $C_{3-7}$ cycloalkylmethyl,
$R^6$ and $R^7$ independently are hydrogen, halogen, alkoxy or trifluoromethyl, and
R" is hydrogen, $C_{1-6}$ alkyl or $C_{3-7}$ cycloalkyl,
for the manufacture of a medicament for use in the treatment of a disorder of the central nervous system.

2. Use according to claim 1, wherein the compound is 3-(5-cyclopropyl-1,2,4-oxadiazol-3-yl)-4,5-dihy-

dro-4-methyl-5-oxo-6-chloroimidazo(1,5-a)quinazoline.

3. A method of preparing a compound of formula I as defined in claim 1, which comprises reacting a compound of formula II

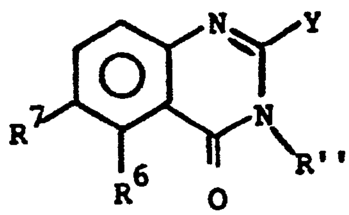

(II)

with a compound of formula III
CN-$CH_2$-X (III)
wherein R", $R^6$, $R^7$ and X are as defined in claim 1 and Y is a leaving group.

4. A method of preparing a compound of formula I as defined in claim 1 when X has definition (i), which comprises reacting a reactive derivative of a compound of formula IV

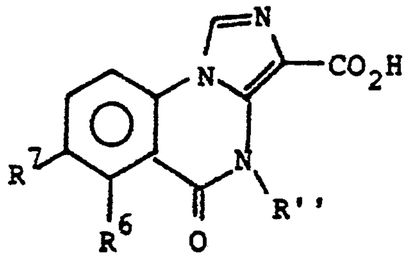

(IV)

with a compound of formula V
R'–C(=NOH)–$NH_2$ (V)
wherein R', R", $R^6$ and $R^7$ are as defined in claim 1.

5. A method of preparing a compound of formula I as defined in claim 1 when X has definition (i), which comprises reacting a compound of formula VI

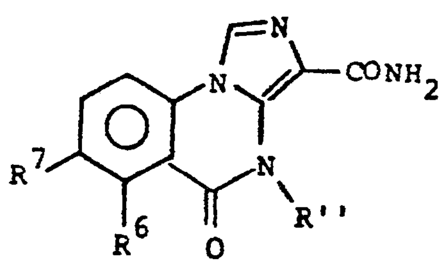

(VI)

with a compound of formula VII
R'–$C(OCH_3)_2N(CH_3)_2$ (VII)
and reacting the resultant compound of formula VIII

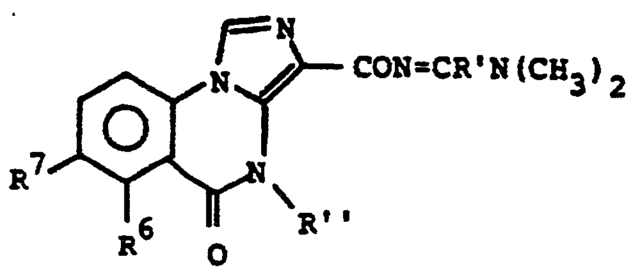

(VIII)

with $NH_2OH$ or another aminating agent, wherein R', R", $R^6$ and $R^7$ are as defined in claim 1.

6. A method of preparing a compound of formula I as defined in claim 1 when X has definition (ii), which comprises reacting a compound of formula IX

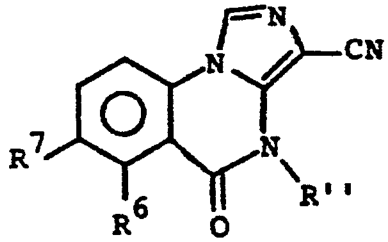

(IX)

with $NH_2OH$, and reacting the resultant compound of formula X

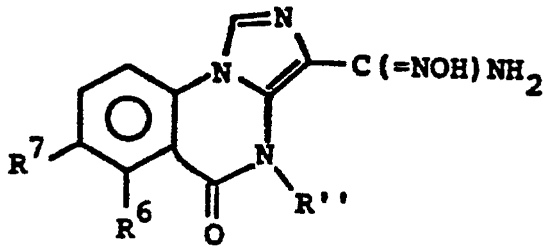

(X)

with R'-COCl, wherein R', R", $R^6$ and $R^7$ are as defined in claim 1.

7. A method according to claim 3, which is carried out under alkaline conditions.

**Patentansprüche für die Vertragsstaaten AT, ES, GR**

1. Verwendung einer Chinazolinverbindung der Formel

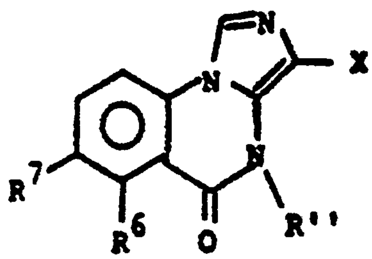

(I)

worin

X (i) 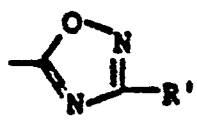 , (ii) 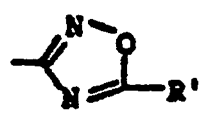 oder (iii) $CO_2R'$ ist

wobei R' $C_{1-6}$-Alkyl, $C_{3-7}$-Cycloalkyl oder $C_{3-7}$-Cycloalkyl-methyl ist,
$R^6$ und $R^7$ unabhängig voneinander Wasserstoff, Halogen, Alkoxy oder Trifluormethyl sind, und
R" Wasserstoff, $C_{1-6}$-Alkyl oder $C_{3-7}$-Cycloalkyl ist,
für die Herstellung eines Arzneimittels zur Verwendung bei der Behandlung einer Störung des Zentralnervensystems.

2. Verwendung nach Anspruch 1, wobei die Verbindung 3-(5-Cyclopropyl-1,2,4-oxadiazol-3-yl)-4,5-dihydro-4-methyl-5-oxo-6-chlorimidazo(1,5-a)chinazolin ist.

3. Ein Verfahren zur Herstellung einer Verbindung der Formel I wie in Anspruch 1 definiert, welches das Umsetzen einer Verbindung der Formel II

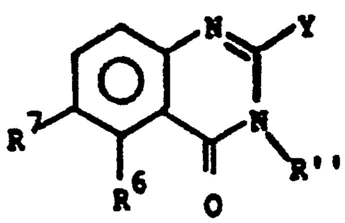

(II)

mit einer Verbindung der Formel III

CN–$CH_2$–X (III),

worin R", $R^6$, $R^7$ und X wie in Anspruch 1 definiert sind, und Y eine austretende Gruppe ist, umfaßt.

4. Verfahren zur Herstellung einer Verbindung der Formel I wie in Anspruch 1 definiert, wenn X die Definition (i) hat, welches das Umsetzen eines reaktiven Derivats einer Verbindung der Formel IV

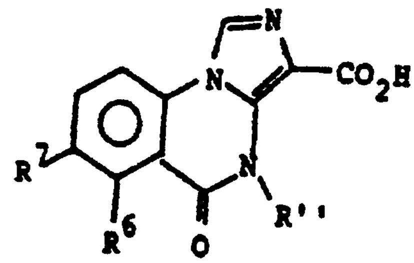

(IV)

mit einer Verbindung der Formel V

R'–C(=NOH)–$NH_2$ (V)

worin R', R", $R^6$ und $R^7$

5. Ein Verfahren zur Herstellung einer Verbindung der Formel I wie in Anspruch 1 definiert, wenn X die Definition (i) hat, welches das Umsetzen einer Verbindung der Formel VI

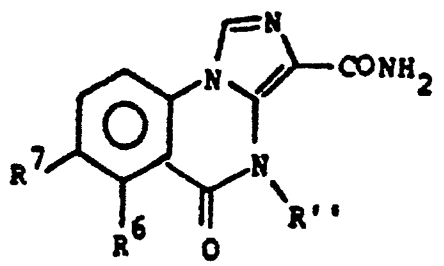

(VI)

mit einer Verbindung der Formel VII

R'–$C(OCH_3)_2$–$N(CH_3)_2$ (VII)

und das Umsetzen der entstandenen Verbindung der Formel VIII

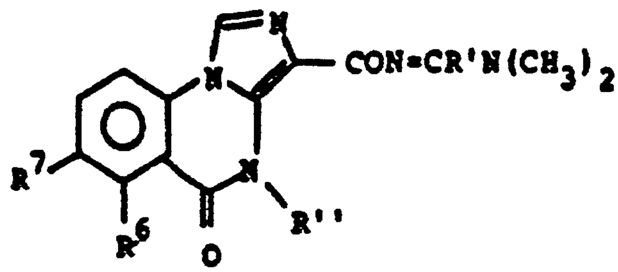

(VIII)

mit $NH_2OH$ oder einem anderen Aminierungsmittel, worin R', R", $R^6$ und $R^7$ wie in Anspruch 1 definiert sind, umfaßt.

6. Ein Verfahren zur Herstellung einer Verbindung der Formel I wie in Anspruch 1 definiert, wenn X die Definition (ii) hat, welches das Umsetzen einer Verbindung der Formel IX

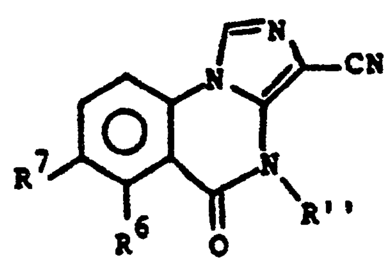

(IX)

mit $NH_2OH$, und das Umsetzen der entstandenen Verbindung der Formel X

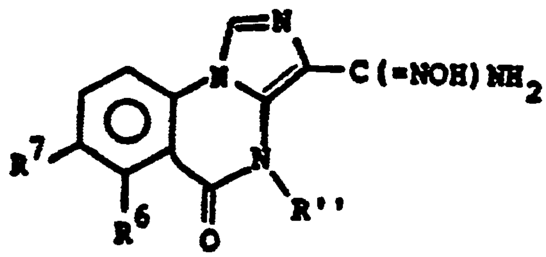

(X)

mit R'–COCl, worin R', R", $R^6$ und $R^7$ wie in Anspruch 1 definiert sind, umfaßt.

7. Ein Verfahren nach Anspruch 3, welches unter alkalischen Bedingungen ausgeführt wird.

**Revendications pour Etats Contractants: AT, ES, GR**

1. Utilisation d'une quinazoline, répondant à la formule

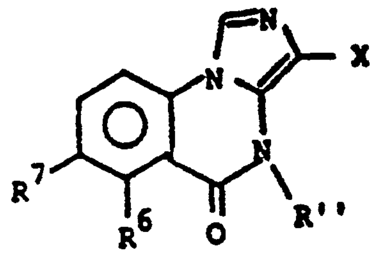

(I)

dans laquelle

X représente (i) 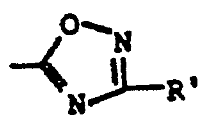 , (ii) 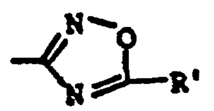 ou (iii) $CO_2R'$,

formules dans lesquelles R′ représente un groupe alkyle en $C_1$ à $C_6$, cycloalkyle en $C_3$ à $C_7$ ou cycloalkylméthyle en $C_3$ à $C_7$,
$R^6$ et $R^7$ représentent, indépendamment l'un de l'autre, un atome d'hydrogène ou d'halogène ou un groupe alcoxy ou trifluorométhyle, et
R" représente un atome d'hydrogène, un groupe alkyle en $C_1$ à $C_6$ ou cycloalkyle en $C_3$ à $C_7$,
pour la fabrication d'un médicament destiné à servir au traitement d'un trouble du système nerveux central.

2. Utilisation selon la revendication 1, dans laquelle le composé est la 3-(5-cyclopropyl-1,2,4-oxadiazol-3-yl)-4,5-dihydro-4-méthyl-5-oxo-6-chloroimidazo(1,5-a)quinazoline.

3. Procédé de préparation d'un composé de formule I, selon la définition donnée à la revendication 1, qui comprend la réaction d'un composé de formule II

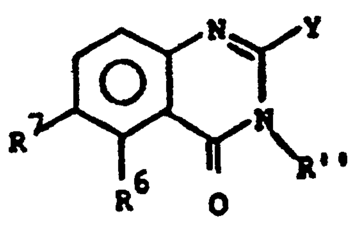

(II)

avec un composé de formule III

$CN-CH_2-X$ (III)

formules dans lesquelles R", $R^6$, $R^7$ et X sont comme définis à la revendication 1, et Y représente un groupe partant.

4. Procédé de préparation d'un composé de formule I, selon la définition donnée à la revendication 1, quand X a la définition (i), qui comprend la réaction d'un dérivé réactif d'un composé de formule IV

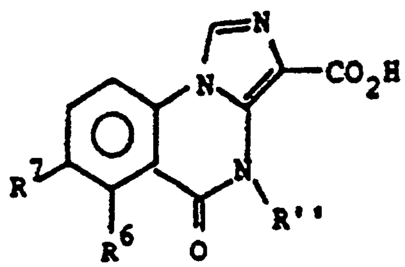

(IV)

avec un composé de formule V

R'–C(=NOH)–$NH_2$ (V)

formules dans lesquelles R', R", $R^6$ et $R^7$ sont comme définis à la revendication 1.

5. Procédé de préparation d'un composé de formule I, selon la définition donnée à la revendication 1, quand X a la définition (i), qui comprend la réaction d'un composé de formule VI

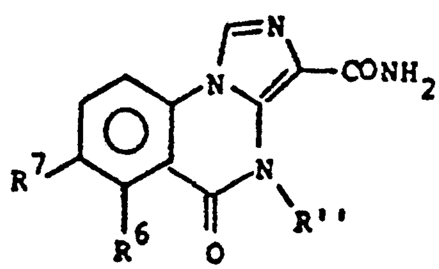

(VI)

avec un composé de formule VII

R'–$C(OCH_3)_2N(CH_3)_2$ (VII)

et la réaction du composé résultant, de formule VIII,

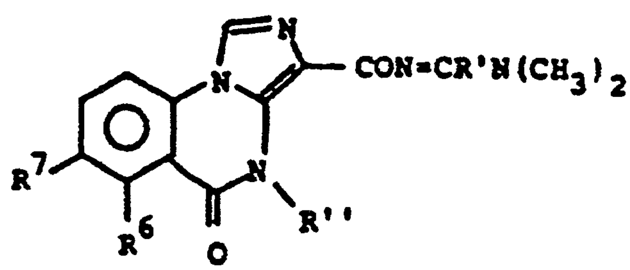

(VIII)

avec $NH_2OH$ ou un autre agent d'amination, formules dans lesquelles R', R", $R^6$ et $R^7$ sont comme définis à la revendication 1.

6. Procédé de préparation d'un composé de formule I selon la définition donnée à la revendication 1, quand X a la définition (i), qui comprend la réaction d'un composé de formule IX

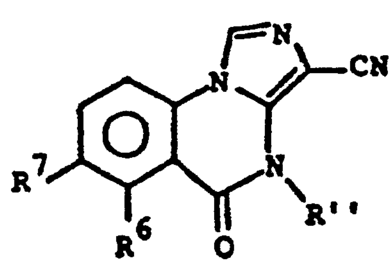

(IX)

avec $NH_2OH$, et la réaction du composé résultant, de formule X

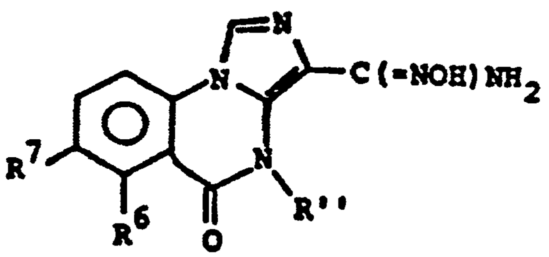

(X)

avec R'–COCl, formules dans lesquelles R', R", $R^6$ et $R^7$ sont comme définis à la revendication 1.

7. Procédé selon la revendication 3, qui est mis en œuvre dans des conditions alcalines.

**Patentansprüche für die Vertragsstaaten BE, CH, DE, FR, GB, IT, LI, LU, NL, SE**

1. Eine Chinazolinverbindung der Formel

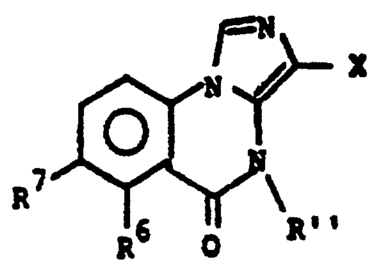

(I)

worin

X (i) 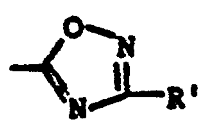 , (ii) 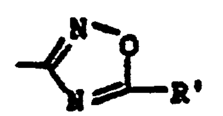 , oder (iii) $CO_2R'$ ist,

wobei R' $C_{1-6}$-Alkyl, $C_{3-7}$-Cycloalkyl oder $C_{3-7}$-Cycloalkyl-methyl ist,
$R^6$ und $R^7$ unabhängig voneinander Wasserstoff, Halogen, Alkoxy oder Trifluormethyl sind, und
R" Wasserstoff, $C_{1-6}$-Alkyl oder $C_{3-7}$-Cycloalkyl ist.

2. Eine Verbindung des Anspruchs 1, welche 3-(5-Cyclopropyl-1,2,4-oxadiazol-3-yl)-4,5-dihydro-4-methyl-5-oxo-6-chlor-imidazo(1,5-a)chinazolin ist.

3. Eine Verbindung des Anspruchs 1 oder des Anspruchs 2 für therapeutische Verwendung.

4. Die Verwendung einer Verbindung des Anspruchs 1 oder des Anspruchs 2 für die Herstellung eines Arzneimittels zur Verwendung bei der Behandlung einer Störung des Zentralnervensystems.

5. Ein Verfahren zur Herstellung einer Verbindung nach Anspruch 1, welches das Umsetzen einer Verbindung der Formel II

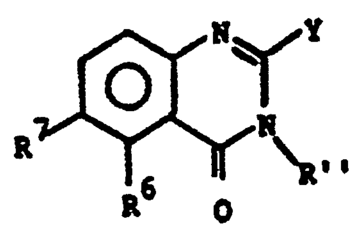

(II)

mit einer Verbindung der Formel III

$CN-CH_2-X$ (III),

worin R", $R^6$, $R^7$ und X wie in Anspruch 1 definiert sind, und Y eine austretende Gruppe ist, umfaßt.

6. Ein Verfahren zur Herstellung einer Verbindung nach Anspruch 1, wenn X die Definition (i) hat, welches das Umsetzen eines reaktiven Derivats einer Verbindung der Formel IV

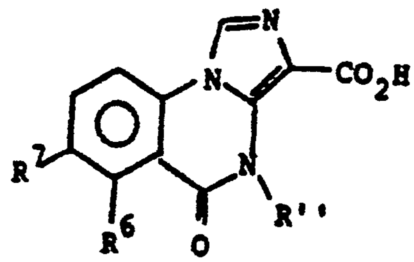

(IV)

mit einer Verbindung der Formel V

R'–C(=NOH)–$NH_2$ (V),

worin R', R", $R^6$ und $R^7$ wie in Anspruch 1 definiert sind, umfaßt.

7. Ein Verfahren zur Herstellung einer Verbindung nach Anspruch 1, wenn X die Definition (i) hat, welches das Umsetzen einer Verbindung der Formel VI

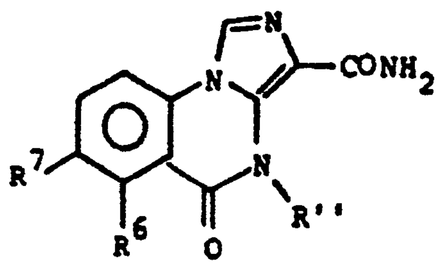

(VI)

mit einer Verbindung der Formel VII

R'–$C(OCH_3)_2$–$N(CH_3)_2$ (VII)

und das Umsetzen der entstandenen Verbindung der Formel VIII

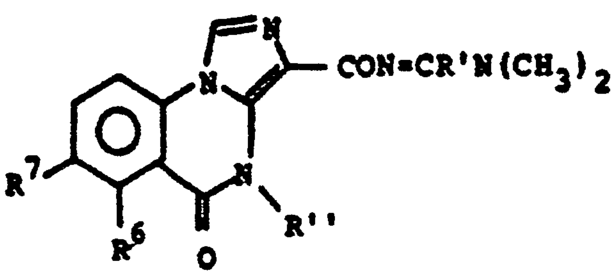

(VIII)

mit $NH_2OH$ oder einem anderen Aminierungsmittel, worin R', R", $R^6$ und $R^7$ wie in Anspruch 1 definiert sind, umfaßt.

8. Ein Verfahren zur Herstellung einer Verbindung nach Anspruch 1, wenn X die Definition (ii) hat, welches das Umsetzen einer Verbindung der Formel IX

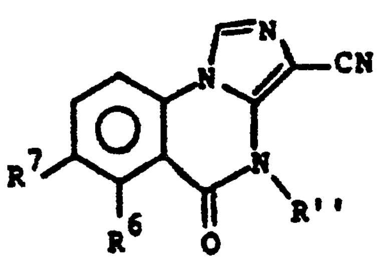

(IX)

mit $NH_2OH$, und das Umsetzen der entstandenen Verbindung der Formel X

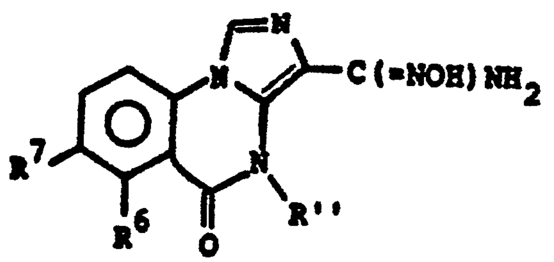

(X)

mit R'–COCl, worin R', R", $R^6$ und $R^7$ wie in Anspruch 1 definiert sind, umfaßt.

9. Ein Verfahren nach Anspruch 5, welches unter alkalischen Bedingungen ausgeführt wird.

**Revendications pour Etats Contractants: BE, CH, DE, FR, GB, IT, LI, LU, NL, SE**

1. Quinazoline, répondant à la formule

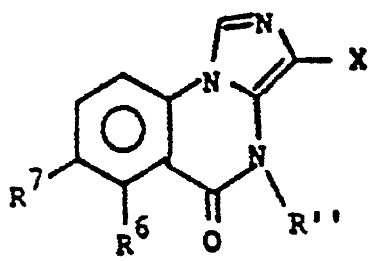

(I)

dans laquelle

X représente (i) 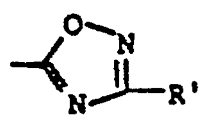 , (ii) 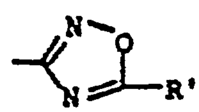 ou (iii) $CO_2R'$

où R' représente un groupe alkyle en $C_1$ à $C_6$, cycloalkyle en $C_3$ à $C_7$ ou cycloalkylméthyle en $C_3$ à $C_7$,
$R^6$ et $R^7$ représentent , indépendamment, chacun un atome d'hydrogène ou d'halogène ou un groupe alcoxy ou trifluorométhyle, et
R" représente un atome d'hydrogène, un groupe alkyle en $C_1$ à $C_6$ ou cycloalkyle en $C_3$ à $C_7$.

2. Composé selon la revendication 1, qui est la 3-(5-cyclopropyl-1,2,4-oxadiazol-3-yl)-4,5-dihydro-4-méthyl-5-oxo-6-chloroimidazo(1,5-a)quinazoline.

3. Composé selon la revendication 1 ou 2, pour utilisation thérapeutique.

4. Utilisation d'un composé selon la revendication 1 ou la revendication 2 pour la fabrication d'un médicament destiné à servir au traitement d'un trouble du système nerveux central.

5. Procédé pour préparer un composé selon la revendication 1, qui comprend la réaction d'un composé de formule II

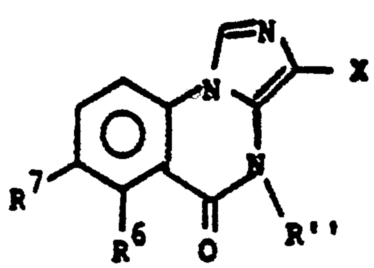

(I)

avec un composé de formule III

$CN-CH_2-X$ (III)

dans laquelle R", $R^6$, $R^7$ et X sont tels que définis à la revendication 1, et Y est un groupe partant.

6. Procédé de préparation d'un composé selon la revendication 1, lorsque X a la définition (i), qui comprend la réaction d'un dérivé réactif d'un composé de formule IV

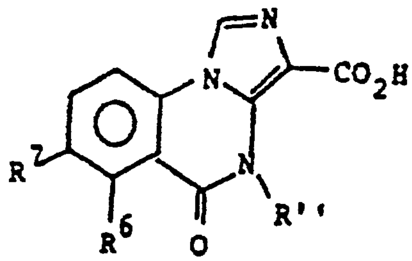

(IV)

avec un composé de formule V

R'–C(=NOH)–$NH_2$ (V)

formules dans lesquelles R', R", $R^6$ et $R^7$ sont comme définis à la revendication 1.

7. Procédé de préparation d'un composé selon la revendication 1, lorsque X a la définition (i), qui comprend la réaction d'un composé de formule VI

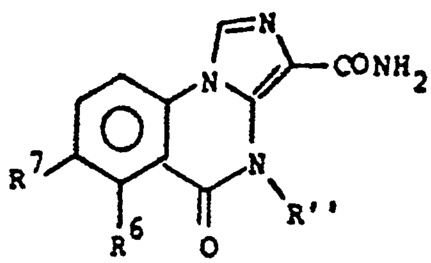

(VI)

avec un composé de formule VII

R'–$C(OC_3)_2$–$N(CH_3)_2$ (VII)

et la réaction du composé résultant, de formule VIII

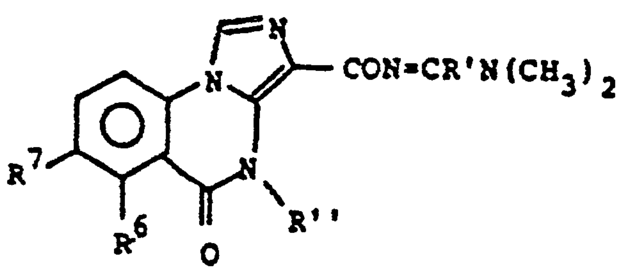

(VIII)

avec $NH_2OH$ ou un autre agent d'amination, formules dans lesquelles R', R", $R^6$ et $R^7$ sont comme définis à la revendication 1.

8. Procédé de préparation d'un composé selon la revendication 1, lorsque X a la définition (ii), qui comprend la réaction d'un composé de formule IX

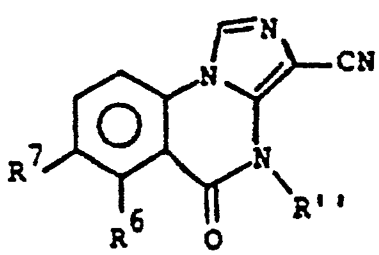

(IX)

avec $NH_2OH$, et la réaction du composé résultant, de formule X

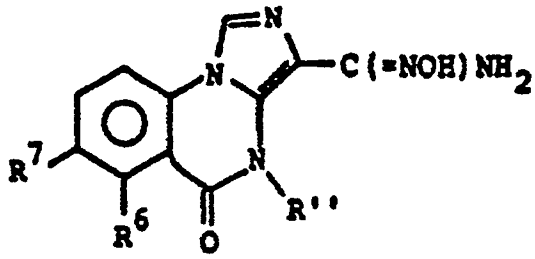

(X)

avec R'–COCl, formules dans lesquelles R', R", $R^6$ et $R^7$ sont comme définis à la revendication 1.

9. Procédé selon la revendication 5, qui est mis en œuvre dans des conditions alcalines.